# EUROPÄISCHE PATENTSCHRIFT

(11) **EP 1 260 179 B1**
(45) Veröffentlichungstag und Bekanntmachung des Hinweises auf die Patenterteilung: **26.03.2003**
(21) Anmeldenummer: 01111988.0
(22) Anmeldetag: 22.05.2001
(51) Int. Cl.: A61B 6/12, A61B 19/00

(54) **Röntgenbildregistrierungseinrichtung mit einem medizinischen Navigationssystem**
X-ray image registration device with a medical navigation system
Appareil pour la régistration d'images radiographiques avec une système de navigation pour applications médicales

(43) Veröffentlichungstag der Anmeldung: 27.11.2002
(73) Patentinhaber: BrainLAB AG, 85551 Kirchheim/Heimstetten (DE)
(72) Erfinder: Schubert, Mario, 85652 Landsham/Pliening (DE); Seifferth, Falko, 85604 Zorneding (DE); Vilsmeier, Stefan, 6330 Kufstein (AT); Zeiss, Mario, 85586 Poing (DE)
(74) Vertreter: Schwabe - Sandmair - Marx

(56) Entgegenhaltungen:
- WO-A-00/56215
- DE-A- 19 917 867
- US-A- 5 446 548
- US-A- 5 799 055

## Beschreibung

Die vorliegende Erfindung betrifft die Registrierung einer Bildinformation. Insbesondere betrifft die Erfindung ein Verfahren zur Registrierung und/oder Neuregistrierung eines Röntgenbildes mittels eines medizinischen Navigationssystems, ein Programm, das, wenn es auf einem Computer läuft oder in einem Computer geladen ist, den Computer veranlasst, das Verfahren durchzuführen, ein Computerprogramm-Speichermedium, welches ein solches Programm aufweist, und eine Röntgenbild-Registrierungseinrichtung.

Während medizinischer Behandlungen, bei denen die Position des Patienten oder von Körperteilen des Patienten mittels eines Navigationssystems getrackt, d. h. bestimmt wird, und bei denen insbesondere auch Instrumente mit dem Navigationssystem getrackt werden, ist es notwendig, eine Registrierung eines Patienten genau zum Zeitpunkt der Bildakquisition, zum Beispiel bei einer Röntgenbildaufnahme durchzuführen, da nicht immer verhindert werden kann, dass der Patient sich bewegt, bewegt wird oder bestimmte zu erfassende Patientenbereiche sich bewegen (zum Beispiel durch Atmung). Eine solche Registrierung wird beispielsweise dadurch bewirkt, dass von dem Patienten ein aktuelles Röntgenbild erstellt wird, welches dann wiederum mit Hilfe von Markierungsanordnungen in dem Navigationssystem zu registrieren ist. Mit dem Röntgenbild und dessen registrierter Position stehen dann aktuelle Informationen über die momentane Patientenpositionierung zur Verfügung und es kann mit solchen aktuellen Daten navigiert und behandelt werden.

Es ist im Zusammenhang mit den obigen Maßnahmen sehr wichtig, zu wissen, zu welchem Zeitpunkt ein Röntgenbild erstellt wurde, um eine Registrierung für den aktuellen Zustand durchzuführen. Bei einem bekannten System, dem sogenannten FluoroNav-System der Firma Sofamor Danek wird ein spezieller röntgensensitiver Sensor verwendet, um die Position des Röntgengerätes, eines C-Bogen-Gerätes und der Patientenmarkeranordnung automatisch zu registrieren, wenn ein Röntgenbild mit dem C-Bogen erstellt wird. Diese Methode ist schon wegen der Notwendigkeit der Bereitstellung eines Sensors und der dazugehörigen Signalübertragung aufwändig, teuer und fehleranfällig.

Eine weitere derzeit verwendete Methode basiert auf einer manuellen Eingabe, d. h. es wird dem Navigations- bzw. Computersystem durch "Knopfdruck" mitgeteilt, dass ein Röntgenbild erstellt wurde und eine Registrierung dieses Röntgenbildes notwendig wird. Auf diese Eingabe hin findet dann durch das Navigationssystem, beispielsweise über Kamerabilder die Registrierung, d. h. die Feststellung der Relativposition des C-Bogens und des Patienten mittels am C-Bogen und am Patienten angebrachte Markeranordnungen statt. siehe z.B. DE 199 17 867.

Dieses Verfahren bringt nachteiligerweise eine Zeitverzögerung zwischen der Erfassung des (neuen) Röntgenbildes mit dem C-Bogen und der tatsächlichen Registrierung durch das Navigationssystem mit sich. Da es möglich ist, dass sich der Patient in dieser Zeitspanne bewegt oder dass seine Markereinrichtung lediglich durch den Atmungsvorgang verschoben wird, kann die Zwischenbilderfassung der Kameras um die bei manueller Registrierung verstrichene Zeit dazu führen, dass eine Ungenauigkeit in der Neuregistrierung vorhanden ist, welche dann auch die Navigation ungenau machen würde.

Zum technischen Hintergrund der Erfindung sind noch die US-Patente US-5,799,055; US-3,577,160; US-5,784,431; US-5,967,982; US-5,772,594 zu nennen, welche sich mit der Röntgenbildregistrierung im Rahmen chirurgischer Navigationsverfahren beschäftigen sowie die US-6,118,845, die ein System und Verfahren zur Reduktion und Eliminierung von Bildstörungen bei der Kalibrierung von Röntgenbilderzeugungsvorrichtungen beschreibt.

Es ist die Aufgabe der vorliegenden Erfindung, die Registrierung einer Bildinformation, insbesondere eines Röntgenbildes im obigen Rahmen derart zu gestalten, dass die erwähnten Nachteile überwunden werden. Bevorzugt soll die Registrierung technisch unaufwändig stattfinden können, und insbesondere soll eine Registrierung mit hoher Genauigkeit ermöglicht werden.

Diese Aufgabe wird erfindungsgemäß gelöst durch ein Verfahren zur Registrierung einer Bildinformation, insbesondere eines Röntgenbildes mittels eines medizinischen Navigationssystems gemäß Anspruch 1.

Wenn im Weiteren Ausführungsformen der Erfindung anhand der Verwendung von Begriffen wie "Röntgenbild" und "Röntgengerät" beschrieben werden, so sind diese Begriffe lediglich als Beispiele für geeignete Bildinformationen und Erfassungsgeräte hierfür zu verstehen, zum Beispiel CT, MR, Ultraschall, SPECT, PET.

Bei dem erfindungsgemäßen Verfahren erkennt also das System computerunterstützt, ob mit einem neuen Röntgenbild auch eine Positionsänderung des Patienten stattgefunden hat. Das Vergleichskriterium ist hierbei der Bildinhaltsunterschied zwischen der aktuellen und einer vorherigen Bildinformation, und es ist von besonderem Vorteil, dass nur dann, wenn tatsächlich eine Bildinhaltsdifferenz vorliegt, eine Neuregistrierung erfolgen muss. Damit wird ein sehr viel aussagekräftigeres Entscheidungskriterium gewählt als beispielsweise in dem Fall, bei dem mittels eines Sensors automatisch jedes Mal dann neu registriert wird, wenn ein Röntgenbild erstellt wird. Außerdem kann das System sehr schnell feststellen, ob ein Bildinhaltsunterschied vorhanden ist, so dass die vorher angesprochene Zeitlücke, d. h., die Zeit die zwischen Bilderstellung und Registrierung bei manueller Eingabe verstreicht, eliminiert und damit die Registrierung sehr genau gemacht werden kann. Natürlich kann auch eine Erst- oder Initial-Registrierung stattfinden. Ein Bildinformationsunterschied ist auch dabei vorhanden, da kein vorheriges Bild vorhanden ist.

Die Erfindung bietet also die Möglichkeit, ohne teuren technischen Aufwand (Sensor und Signalübertragung) eine genaue Registrierung durchzuführen.

Bei einer bevorzugten Ausführungsform des erfindungsgemäßen Verfahrens nimmt das Navigationssystem die Registrierung und Neuregistrierung des Patienten und/oder des Röntgengerätes durch kameragestützte Erfassung von am Patienten bzw. am Röntgengerät angebrachten Markierungen, insbesondere Reflexionsmarkeranordnungen vor.

Vorzugsweise erfolgt die Registrierung rein softwareseitig im Navigationssystem und, wie oben schon angedeutet, erfolgt der Vergleich der Bildinhalte und auch die Registrierung automatisch und ohne Bedieneranforderung.

Die Erfindung betrifft ferner ein Programm gemäß Anspruch 6 und lesbares ein Computer Speichermedium, wie zum Beispiel eine CD, eine DVD oder eine Diskette gemäß Anspruch 7.

Gemäß einem weiteren Aspekt der Erfindung betrifft diese eine Röntgenbild-Registrierungseinrichtung mit einem medizinischen Navigationssystem gemäß Anspruch 8.

Es ist im Rahmen der Erfindung durchaus auch denkbar, anstatt eines Röntgenbildes ein Bild eines Ultraschallgerätes zu verwenden und dieses Bild bzw. diese Bilder in entsprechender Weise zu registrieren. Auch andere Durchleuchtungs- oder Bilderzeugungsverfahren können verwendet werden.

Die Erfindung wird im Weiteren anhand eines beispielhaft aufgezeigten Verfahrensablaufes näher erläutert, wobei auf das beiliegende Ablaufdiagramm Bezug genommen wird.

### Die Situation ist die Folgende:

Ein Patient, an dem in der Umgebung des zu behandelnden Bereiches eine Markierungsanordnung angebracht ist, liegt auf einer Patientenliege und soll behandelt werden, wobei die Behandlung durch ein Navigationssystem unterstützt wird, das die Position des Patienten mit Hilfe der vorgenannten Markeranordnung, die Position von Behandlungsinstrumenten bzw. Behandlungsgeräten, und insbesondere die Position eines C-Bogen-Röntgengerätes erfasst und trackt, und zwar wiederum über Markeranordnungen, die an diesen Instrumenten bzw. Geräte befestigt sind.

Nunmehr wird zunächst eine Registrierung durchgeführt, d. h. die Position des Patienten relativ zur Röntgenaufnahme wird erfasst. Die Position des Patienten und des C-Bogen-Röntgengerätes wird kontinuierlich erfasst. Daraufhin wird ein Röntgenbild gemacht, und die gesamte Situation wird über ein Videobild dargestellt. Hierzu dient eine dem Navigationssystem zugeordnete Video-Einheit, mit der zusätzliche Positionsinformationen erhalten werden können. Das Videobild mit dem C-Bogen-Röntgengerät wird ebenfalls durch das Navigationssystem erfasst, und diese Information wird verwendet, um die Position des Röntgenbildes im 3D-Raum zu errechnen und die Navigation unter Einbeziehung der Röntgenbildinformation zu ermöglichen.

Sind nun die obigen Informationen alle vorhanden, kann es während einer Behandlung einmal oder auch öfter notwendig sein, eine Registrierung vorzunehmen, zum Beispiel um Patientenbewegungen zu kompensieren. Dazu wird ein neues Röntgenbild erstellt, und zu diesem Zeitpunkt greift die vorliegende Erfindung ein. Das Röntgenbild wird, wenn es eine ausreichende Bildqualität aufweist, mittels einer Bildverarbeitung, die im Computer des Navigationssystems durchgeführt wird, mit der vorher vorhandenen Bildinformation verglichen (Software-Vergleich). Wenn dabei ein Bildinhaltsunterschied festgestellt wird, speichert das System automatisch die Position des C-Bogens und des Patienten, die dem Navigationssystem über die Erfassung der angebrachten Markierungen zu jedem Zeitpunkt bekannt ist. Dann kann mit der Behandlung fortgefahren werden, wobei das System die gespeicherte neue Position von C-Bogen und Patienten für die Röntgenbild-Registrierung verwendet.

Wenn im oben beschriebenen Entscheidungsschritt kein Bildinhaltsunterschied festgestellt wird, kann die Registrierung auch manuell nach der bisherigen Methode durchgeführt werden. Das System verwendet die zu diesem Zeitpunkt bestehende Position von C-Bogen und Patientenmarkierung für die Bildregistrierung.

Das System hat also die Position von C-Bogen und Patient gespeichert und wird eine hochgenaue Position für das Röntgenbild im 3D-Raum errechnen, sogar, wenn der Patient oder der C-Bogen bewegt worden sind, und dies gestattet wiederum eine hochgenaue Navigation.

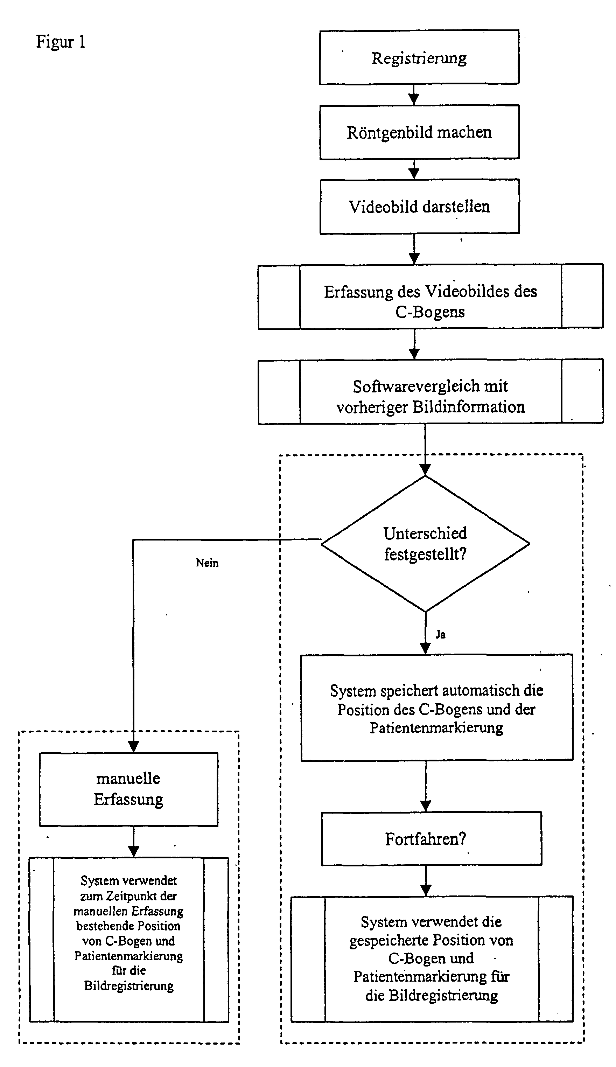

## Patentansprüche

1. Verfahren zur Registrierung bzw. Neuregistrierung der Position der Patienten relativ zu einer Bildinformation mittels eines medizinischen Navigationssystems, bei dem
- eine aktuelle Bildinformation, insbesondere ein aktuelles Röntgenbild eines mit dem Navigationssystem zu registrierenden Patienten erstellt wird,
- die Position des Bildinformations-Erfassungsgerätes, insbesondere des Röntgengeräts mittels des Navigationssystems festgestellt wird,
- die aktuelle Bildinformation, insbesondere das aktuelle Röntgenbild automatisch mit einer anderen Bildinformation (zum Beispiel einem vorher aufgenommenen Röntgenbild) computerunterstützt verglichen wird,
- bei einem Unterschied zwischen dem Bildinhalt des aktuellen und des vorherigen Bildes die neue Relativposition von Bildinformations-Erfassungsgerät bzw. Röntgengerät und Patient automatisch erfasst und gespeichert wird, und
- auf der Basis der Information über die neue Relatiyposition eine Registrierung der erstellten aktuellen Bildinformation bzw. des erstellten aktuellen Röntgenbildes automatisch im Navigationssystem erfolgt.

2. Verfahren nach Anspruch 1, bei dem kontinuierlich im Verlauf der Röntgenerfassung des Patienten eine Speicherung von Patienten- und C-Bogen-Position erfolgt, wenn der Bildinhaltsunterschied festgestellt wird, während sie unterbleibt, wenn ein solcher Unterschied nicht festgestellt wird, wobei dann mit den zu einem späteren Zeitpunkt bekannten Daten weitergearbeitet (registriert) wird.

3. Verfahren nach Anspruch 1 oder 2, bei dem das Navigationssystem die Registrierung und Neuregistrierung des Patienten durch kameragestützte Erfassung von am Patienten bzw. am Röntgengerät angebrachten Markierungen, insbesondere Reflexionsmarkeranordnungen vornimmt.

4. Verfahren nach einem der Ansprüche 1 bis 3, bei dem die Registrierung rein softwareseitig im Navigationssystem erfolgt.

5. Verfahren nach einem der Ansprüche 1 bis 4, bei dem sowohl der Vergleich der Bildinhalte als auch die Registrierung automatisch und ohne Bedieneranforderung stattfindet.

6. Programm, das, wenn es auf einem Computer läuft oder in einem Computer geladen ist, den Computer veranlasst, die zur automatischen Durchführung eines Verfahrens nach einem der Ansprüche 1-5 benötigten Computerbefehle durchzuführen.

7. Computerlesbares Speichermedium, welches die zur automatischen Durchführung eines Verfahrens nach einem der Ansprüche 1-5 benötigten Computerbefehle aufweist.

8. Röntgenbild-Registrierungseinrichtung mit einem medizinischen Navigationssystem, einem Röntgengerät, mit dem ein aktuelles Röntgenbild und die position des Patienten relativ zu Möntgenbild eines mit dem Navigationssystem zu registrierenden Patienten erstellt wird, wobei das Navigationssystem eine Einrichtung umfasst, mit der die Position des Röntgengeräts und die position des Patienten relativ zu Möntgenbild festgestellt wird, **gekennzeichnet durch** eine Bildvergleichseinrichtung, mit der das aktuelle Röntgenbild mit einer vorherigen Bildinformation computerunterstützt automatisch verglichen wird, sowie **durch** eine Erfassungs- und Speichereinrichtung, mit der bei einem Unterschied zwischen dem Bildinhalt der aktuellen und der vorherigen Bildinformation die neue Relativposition von Röntgengerät Röntgenbild und Patient automatisch erfasst und gespeichert wird, wobei auf der Basis der Information über die neue Relativposition eine Neuregistrierung des erstellten aktuellen Röntgenbildes im Navigationssystem automatisch erfolgt.

## Claims

1. A method for registering and/or re-registering the position of the patient relative to image information by means of a medical navigation system, wherein:
- current image information, in particular a current x-ray image of a patient to be registered using the navigation system, is produced;
- the position of the image information detection device, in particular of the x-ray device, is established by means of the navigation system;
- the current image information, in particular the current x-ray image, is automatically compared with the aid of a computer with other image information (for example, a previously taken x-ray image);
- in the event of a difference between the image content of the current and previous image, the new relative position of the image information detection device or x-ray device respectively is automatically detected and stored; and
- the current image information produced or current x-ray image produced respectively is automatically registered in the navigation system, based on the information on the new relative position.

2. The method as set forth in claim 1, wherein the position of the C-arc and patient is continuously stored in the course of x-ray detecting the patient, when a difference in the image content is established, while this does not occur when such a difference is not established, work (registering) then continuing using the image data known at a later point in time.

3. The method as set forth in claim 1 or 2, wherein the navigation system registers and re-registers the patient by detecting markings arranged on the patient or the x-ray device respectively, in particular arrangements of reflection markers, with the aid of cameras.

4. The method as set forth in any one of claims 1 to 3, wherein registering in the navigation system takes place purely on the software side.

5. The method as set forth in any one of claims 1 to 4, wherein comparison of the image contents and registering take place automatically and without prompting by the operator.

6. A program which, when run on a computer or loaded in a computer, causes the computer to carry out the computer commands required to automatically carry out a method in accordance with any one of claims 1 to 5.

7. A computer-readable storage medium comprising the computer commands required to automatically carry out a method in accordance with any one of claims 1 to 5.

8. An x-ray image registering means comprising a medical navigation system, an x-ray device with which a current x-ray image is produced of a patient to be registered using the navigation system, the navigation system including a means by which the position of the x-ray device and the position of the patient relative to the x-ray image is established, **characterised by** an image comparing means with which the current x-ray image is automatically compared with the aid of computers with previous image information, as well as by a detecting and storing means with which, in the event of a difference between the image content of the current and previous image information, the new relative position of the x-ray device, x-ray image and the patient is automatically detected and stored, wherein the current x-ray image produced is automatically re-registered in the navigation system, based on the information on the new relative position.

## Revendications

1. Procédé d'enregistrement ou de réenregistrement de la position du patient relativement à une information d'image au moyen d'un système de navigation pour applications médicales, pour lequel
- une information d'image actuelle, en particulier une image radiographique actuelle d'un patient à enregistrer avec le système de navigation est réalisée,
- la position de l'appareil de saisie de l'information d'image, en particulier de l'appareil à rayons x, est déterminée au moyen du système de navigation,
- l'information d'image actuelle, en particulier l'image radiographique actuelle, est automatiquement comparée avec une autre information d'image (par exemple une image radiographique préalablement réalisée) avec l'assistance de l'ordinateur,
- en cas de différence entre le contenu de l'image actuelle et de l'image précédente, la nouvelle position relative de l'appareil de saisie de l'information d'image ou de l'appareil à rayons X et du patient est automatiquement saisie et enregistrée, et
- sur base de l'information sur la nouvelle position relative, un enregistrement de l'information d'image actuelle réalisée ou de l'image radiographique actuelle réalisée est automatiquement effectué dans le système de navigation.

2. Procédé suivant la revendication 1, pour lequel un enregistrement de la position du patient et de l'appareil à rayons X à courbure en C est réalisé en continu au cours de l'imagerie à rayons X du patient si la différence de contenu d'image est constatée, tandis qu'il est annulé si une telle différence n'est pas constatée, le travail (l'enregistrement) étant alors poursuivi avec les données connues à un moment ultérieur.

3. Procédé suivant la revendication 1 ou 2, pour lequel le système de navigation effectue l'enregistrement et le réenregistrement du patient par détection assistée par caméra de marqueurs fixés sur le patient ou sur l'appareil à rayons X, en particulier de dispositifs marqueurs à réflexion.

4. Procédé suivant l'une des revendications 1 à 3, pour lequel l'enregistrement s'effectue dans le système de navigation de manière purement logicielle.

5. Procédé suivant l'une des revendications 1 à 4, pour lequel tant la comparaison des contenus d'image que l'enregistrement ont lieu automatiquement et sans intervention de l'opérateur.

6. Programme qui, lorsqu'il tourne sur un ordinateur ou est chargé dans un ordinateur, amène l'ordinateur à exécuter les commandes d'ordinateur requises pour la réalisation automatique d'un procédé suivant l'une des revendications 1-5.

7. Support d'information pouvant être lu par un ordinateur qui présente les commandes d'ordinateur requises pour la réalisation automatique d'un procédé suivant l'une des revendications 1-5.

8. Appareil pour l'enregistrement d'images radiographiques avec un système de navigation pour applications médicales, un appareil à rayons X, avec lequel une image radiographique actuelle et la position du patient relativement à l'image radiographique d'un patient à enregistrer avec le système de navigation sont élaborées, le système de navigation comprenant un dispositif avec lequel la position de l'appareil à rayons X et la position du patient relativement à l'appareil à rayons X sont déterminées, **caractérisé par** un dispositif de comparaison d'image avec lequel l'image radiographique actuelle est automatiquement comparée avec une information d'image précédente avec l'assistance de l'ordinateur, ainsi que par un dispositif de saisie et d'enregistrement avec lequel, en cas de différence entre le contenu d'image de l'information d'image actuelle et de l'information d'image précédente, la nouvelle position relative de l'appareil à rayons X, de l'image radiographique et du patient est automatiquement saisie et enregistrée, un réenregistrement de l'image radiographique actuelle réalisée étant automatiquement effectué dans le système de navigation sur base de l'information sur la nouvelle position relative.
